(19) Europäisches Patentamt
European Patent Office
Office européen des brevets

(11) Veröffentlichungsnummer: **0 029 407**
**A2**

(12) # EUROPÄISCHE PATENTANMELDUNG

(21) Anmeldenummer: **80810253.7**

(22) Anmeldetag: **18.08.80**

(51) Int. Cl.³: **C 07 D 249/12**, A 01 N 47/38

(30) Priorität: **21.08.79 CH 7625/79**
**26.06.80 CH 4912/80**

(43) Veröffentlichungstag der Anmeldung: **27.05.81**
**Patentblatt 81/21**

(84) Benannte Vertragsstaaten: **AT BE CH DE FR GB IT LI NL**

(71) Anmelder: **CIBA-GEIGY AG, Patentabteilung Postfach, CH-4002 Basel (CH)**

(72) Erfinder: **Kristiansen, Odd, Dr., Delligrabenstrasse 7, CH-4313 Möhlin (CH)**
Erfinder: **Drabek, Jozef, Dr., Benkenstrasse 12, CH-4104 Oberwil (CH)**

(54) **1-N,N-Dimethylcarbamoyl-3(5)-alkyl-5(3)-alkoxyalkylthio-1,2,4-triazole, Verfahren zu ihrer Herstellung, diese Verbindungen enthaltende Mittel und ihre Verwendung zur Bekämpfung von Schädlingen sowie ihre Ausgangsprodukte und deren Herstellung.**

(57) Verbindung der Formel IA bzw. IB

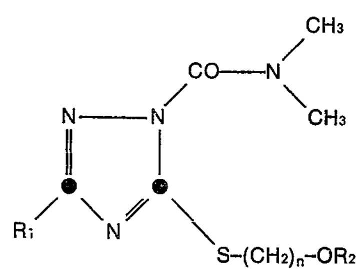

(IA)

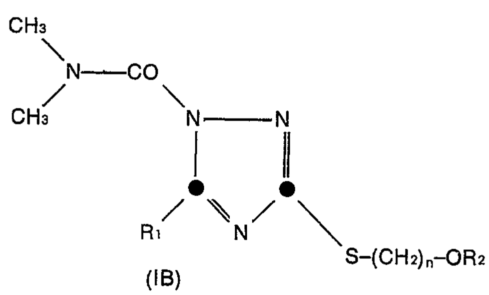

(IB)

worin $R_1$ i-Propyl, s-Butyl, t-Butyl oder gegebenenfalls durch Methyl substituiertes Cyclopropyl, $R_2$ $C_1$-$C_3$-Alkyl und n die Zahl 1 oder 2 bedeuten sind neu. Die Verbindungen sind pestizid vor allem insektizid wirksam.

EP 0 029 407 A2

0029407

CIBA-GEIGY AG
Basel (Schweiz)

5-12485/1+2

BEZEICHNUNG GEÄNDERT
siehe Titelseite

1,2,4-Triazole mit pestizider Wirkung

Die vorliegende Erfindung betrifft neue 1-N,N-Dimethylcarbamoyl-3(5)-alkyl-5(3)-alkoxyalkylthio-1,2,4-triazole, welche wirksam gegen Schädlinge sind, ein Verfahren zur Herstellung dieser Verbindungen, Schädlingsbekämpfungsmittel, welche die neuen Verbindungen als aktive Komponente enthalten, sowie die Bekämpfung von Schädlingen unter Verwendung der genannten Triazole.

1-N,N-Dialkylcarbamoyl-3(5)-alkyl-5(3)-hydrocarbylthio-1,2,4-triazole mit pestizider, vor allem insektizider, Wirkung sind bekannt (siehe z.B. US Patent Nr. 3,308,131 und 4,066,774, sowie britisches Patent Nr. 1,510,636). Nach vorliegender Erfindung werden neuartige Verbindungen dieses Typs vorgeschlagen, die überraschenderweise eine besonders gute Wirkung gegen Insekten besitzen und welche aufgrund ihrer vorteilhaften biologischen Eigenschaften für die praktische Anwendung besonders geeignet sind.

Die erfindungsgemässen, neuen 1-N,N-Dimethylcarbamoyl-3(5)-alkyl-5(3)-alkoxyalkylthio-1,2,4-triazole entsprechen der Formel IA bzw. IB

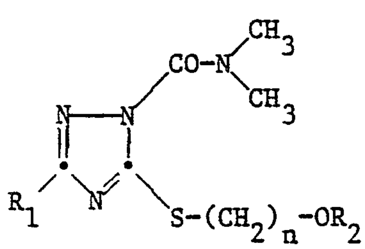

(IA)

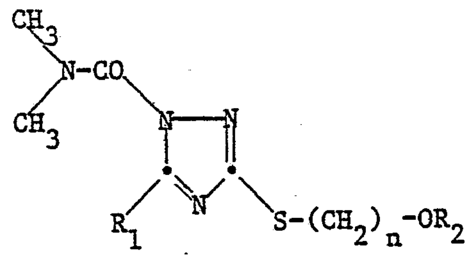

(IB)

worin $R_1$ eine i-Propyl-, s-Butyl-, t-Butyl- oder eine gegebenenfalls durch Methyl substituierte Cyclopropylgruppe, $R_2$ eine $C_1$-$C_3$-Alkylgruppe und n die Zahl 1 oder 2 bedeuten.

Die für $R_2$ in Frage kommenden Alkylgruppen sind die Methyl-, Aethyl-, n-Propyl- und i-Propylgruppe.

Wegen ihrer biologischen Wirkung sind besonders die Verbindungen der Formeln IA und IB von Interesse, worin $R_1$ ein i-Propylgruppe, Cyclopropylgruppe oder t-Butylgruppe und $R_2$ eine Methyl- oder Aethylgruppe bedeuten. Aufgrund ihrer guten pestiziden Eigenschaften sind vor allem solche Verbindungen der Formeln IA und IB hervorzuheben, worin $R_1$ für eine i-Propylgruppe und $R_2$ für eine Methylgruppe steht.

Die erfindungsgemässen Verbindungen können in Form von Isomeren der oben dargestellten Formel IA und IB vorliegen. Nach dem hierin beschriebenen Herstellungsverfahren werden Gemische dieser zwei Isomeren erhalten, wobei in gewissen Fällen (z.B. bei Verbindungen, worin $R_1$ t-Butyl oder 1- bzw. 2-Methyl-cyclopropyl bedeutet) der überwiegende Anteil des Gemisches bzw. annähernd das ganze Produkt aus dem 3-Alkyl-5-alkoxyalkylthio-Isomeren der Formel IA besteht.
Solche Isomerengemische können nach bekannten Methoden (z.B. durch chromatographische Trennung) in die einzelnen isomeren Formen aufgetrennt werden. Die beiden Isomeren der Formel IA und IB werden jedoch zweckmässig stets in Form ihrer nach dem beschriebenen Herstellungsverfahren hergestellten, unaufgetrennten Gemische verwendet. Unter dem Begriff der gegenwärtigen Erfindung sind

demzufolge sowohl die einzelnen Isomeren der Formel IA und IB als auch Gemische derselben zu verstehen.

Die erfindungsgemässen Verbindungen der Formel IA und IB zeichnen sich durch eine ausgezeichnete insektizide Wirkung aus. Insbesondere weisen die erwähnten Verbindungen sowohl eine sehr gute systemische als auch Kontakt-Wirkung gegen saugende Insekten, z.B. der Ordnung Homoptera und von allem der Familie Aphididae (wie z.B. Aphis fabae, Aphis craccivora und Myzus persicae) auf.

Darüber hinaus zeigen die erfindungsgemässen Verbindungen auch eine Wirkung gegen pflanzenschädigende Akariden (Milben) z.B. der Familien Tetranychidae und Tyroglyphidae.

Die Verbindungen der Formel IA und IB sind demzufolge erfindungsgemäss zur Bekämpfung von pflanzenschädigenden Insekten in Kulturen von Nutz- und Zierpflanzen, vor allem in Baumwolle-, Obst- und Gemüsekulturen, besonders geeignet.

Die Verbindungen der Formel IA und IB werden analog bekannten Verfahren hergestellt, in dem man z.B. eine Verbindung der Formel II

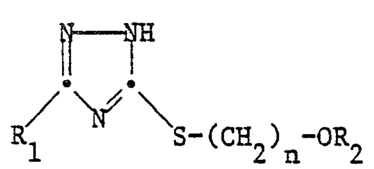

(II)

worin $R_1$, $R_2$ und n die für Formel IA und IB bereits angegebenen Bedeutungen haben in Gegenwart einer Base mit einem N,N-Dimethylcarbamoylhalid, insbesondere N,N-Dimethylcarbamoylchlorid reagieren lässt.

Das Verfahren wird zweckmässig bei einer Temperatur zwischen 10° und 100°C, meist zwischen 40° und 80°C, bei normalem oder leicht erhöhtem Druck und vorzugsweise in Gegenwart eines gegenüber den Reaktionsteilnehmern inerten Lösungs- oder Verdünnungsmittels durchgeführt.

Als Lösungs- oder Verdünnungsmittel eignen sich z.B. Ketone wie Aceton, Methyläthylketon und Cyclohexanon sowie Acetonitril.

Als für diese Verfahren geeignete Basen kommen anorganische und organische Basen, insbesondere tertiäre Amine, wie Trialkylamine, Pyridine und Dialkylaniline; ferner Hydroxide, Oxide, Carbonate und Bicarbonate von Alkali- und Erdalkalimetallen; sowie Alkalimetallalkoholate wie z.B. Kalium-tert.-butylat und Natrium-methylat in Betracht.

Die Ausgangsstoffe der Formel II sind neu und gehören ebenfalls zum Erfindungsgegenstand. Sie können analog bekannten Methoden aus bereits vorhandenen Vorläufern dadurch erhalten werden, indem man z.B. eine Verbindung der Formel III

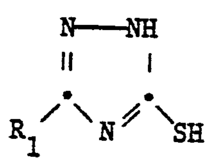

(III)

in Gegenwart einer Base (wie z.B. $NaOC_2H_5$) mit einer Verbindung der Formel IV

$$Hal-(CH_2)_n-OR_2 \qquad (IV)$$

umsetzt, wobei in den Formeln III und IV, $R_1$, $R_2$ und n die unter Fomel I bereits angegebenen Bedeutungen haben und "Hal" ein Halogenatom bedeutet.

Das Verfahren zur Herstellung des Ausgangsstoffes der Formel III wird zweckmässig bei einer Temperatur zwischen 50 und 100°C und vorzugsweise in Gegenwart eines gegenüber den Reaktionsteilnehmern inerten Lösungs- oder Verdünnungsmittels, wie z.B. Aethanol, durchgeführt.

Die Verbindungen der Formel IA und IB und deren Gemische werden erfindungsgemäss als solche verwendet oder bilden einen Bestandteil von Mitteln, welche noch geeignete Trägerstoffe oder Zuschlagstoffe oder Gemische solcher Stoffe enthalten.

Geeignete Träger und Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Binde- und/oder Düngemitteln.

Die insektizide bzw. akarizide Wirkung der erfindungsgemässen Mittel lässt sich durch Zusatz anderer Akarizide und/oder Insektizide wesentlich verbreitern.

Als Zusätze eignen sich z.B.: org. Phosphorverbindungen; Nitrophenole und deren Derivate; Formamidine; Harnstoffe; pyrethrinartige Verbindungen; Karbamate und chlorierte Kohlenwasserstoffe.

Die erfindungsgemässen Mittel können z.B. als Stäubemittel, Dispersionen, Lösungen und Aufschlämmungen sowie als in Wasser dispergierbare Spritzpulver, Pasten, Emulsionen und Emulsionskonzentrate vorliegen und angewendet werden. Vorzugsweise aber werden die genannten Mittel als Granulate (z.B. Umhüllungsgranulate, Imprägnierungsgranulate und Homogengranulate), welche zum Streuen auf die Bodenoberfläche besonders geeignet sind, formuliert.

Der Gehalt an Wirkstoff in den oben beschriebenen Mitteln liegt zwischen 0,1 bis 95%, dabei ist zu erwähnen, dass bei der Applikation aus dem Flugzeug oder mittels anderer geeigneten Applikationsgeräte auch höhere Konzentrationen eingesetzt werden können.

Die erfindungsgemässen Verbindungen (Wirkstoffe) können beispielsweise wie folgt formuliert werden:

Granulat: Zur Herstellung eines 5%igen Granulates werden die folgenden Stoffe verwendet:

| | |
|---|---|
| 5 | Teile Wirkstoff, |
| 0,25 | Teile epoxydiertes Pflanzenöl, |
| 0,25 | Teile Cetylpolyglykoläther |
| 3,50 | Teile Polyäthylenglykol, |
| 91 | Teile Kaolin (Korngrösse 0,3 - 0,8 mm). |

Die Aktivsubstanz wird mit dem epoxydierten Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Emulsionskonzentrat I

20 Gew.-Teile des obengenannten Wirkstoffes werden in
70 Gew.-Teilen Xylol gelöst und mit
10 Gew.-Teilen eines Emulgiermittels, bestehend aus einem Gemisch eines Arylphenylpolyglykoläthers und dem Calciumsalz der Dodecylbenzolsulfonsäure, versetzt. Das Emulsionskonzentrat kann in beliebigem Verhältnis mit Wasser versetzt werden und bildet dabei eine milchige Emulsion.

Emulsionskonzentrat II

5 bis max. 30 Gew.-Teile Wirkstoff werden unter Rühren bei Zimmertemperatur in 30 Gew.-Teilen Dibutylnaphthalat, 10 Gew.-Teilen Solvent 200 (niederviskoses hocharomat. Erdöldestillat) und 15 bis 35 Gew.-Teilen Dutrex 238 CF (viskoses hocharomat. Erdöldestillat) gelöst und mit 10 Gew.-Teilen eines Emulgatorgemisches, bestehend aus Ricinusölpolyglykoläther und dem Calciumsalz der Dodecylbenzolsulfonsäure, versetzt. Das so erhaltene Emulsionskonzentrat gibt in Wasser milchige Emulsionen.

Spritzpulver

5 bis 30 Gew.-Teile des Wirkstoffes werden in einer Mischapparatur mit
5 Gew.-Teilen eines aufsaugenden Trägermaterials (Kieselsäure K 320 oder Wessalon S) und
55 bis 80 Gew.-Teilen eines Trägermaterials (Bolus alba oder Kaolin B 24) und einem Dispergiermittelgemisch, bestehend aus
5 Gew.-Teilen eines Na-lauryl-sulfonates und
5 Gew.-Teilen eines Alkyl-aryl-polyglykoläthers, intensiv vermischt. Diese Mischung wird auf einer Stift- oder Luftstrahlmühle bis auf 5-15 µm gemahlen. Das so erhaltene Spritzpulver gibt in Wasser eine gute Suspension.

Stäubemittel

2 Gew.-Teile feingemahlener Wirkstoff werden mit
1 Gew.-Teil einer gefällten Kieselsäure und
97 Gew.-Teilen Talk intensiv vermischt.

Beispiel 1: Herstellung von 1-N,N-Dimethylcarbamoyl-3(5)-i-propyl-5(3)-(2-äthylthio-äthylthio)-1,2,4-triazol

a) Herstellung des Ausgangsstoffes:

Zu einer Lösung von 14,3 g 3-i-Propyl-5-merkapto-1,2,4-triazol in äthanolischem Natriumäthylat (2,3 g Natrium in 150 ml Aethanol) wurde 10,9 g 2-Chlordiäthyläther getropft. Das Reaktionsgemisch wurde 6 Stunden unter Rückfluss gekocht und nach dem Abkühlen abfiltriert. Anschliessend wurde das Lösungsmittel unter Vakuum abgetrieben und der Rückstand in 300 ml Chloroform aufgenommen und mit 100 ml Wasser ausgeschüttelt. Nach dem Abdestillieren des Lösungsmittels und Waschen des Rückstandes mit Petroläther erhielt man das 3-i-Propyl-5-(2-äthyloxy-äthylthio)-1,2,4-triazol der Formel

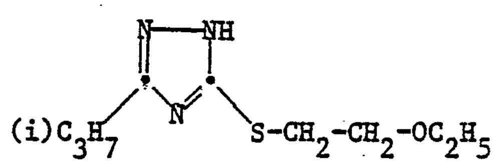

mit einem Schmelzpunkt von 56 - 58°.

Die nachfolgenden Verbindungen der Formel II

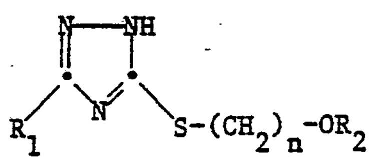

(II)

sind auf analoge Weise erhältlich:

| $R_1$ | $R_2$ | n | Physikalische Daten |
|---|---|---|---|
| $(i)C_3H_7$ | $CH_3$ | 1 | |
| $(i)C_3H_7$ | $CH_3$ | 2 | Smp. 48 - 50° |
| $(i)C_3H_7$ | $C_2H_5$ | 1 | |
| $(t)C_4H_9$ | $CH_3$ | 2 | Smp. 92 - 94° |
| $(t)C_4H_9$ | $C_2H_5$ | 1 | |
| $(t)C_4H_9$ | $C_2H_5$ | 2 | Harz |
| $(t)C_4H_9$ | $(n)C_3H_7$ | 2 | |
| $(s)C_4H_9$ | $C_2H_5$ | 2 | |
| 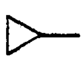 | $C_2H_5$ | 2 | |
| 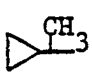 | $C_2H_5$ | 2 | Smp. 68 - 74° |
| 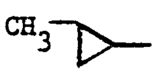 | $C_2H_5$ | 2 | |

b) Herstellung des Endproduktes:

Zu einer Suspension von 12,3 g 3-i-Propyl-5-(2'-äthoxyäthylthio)-1,2,4-triazol und 8 g wasserfreiem Kaliumcarbonat in 150 ml Acetonitril wurde 7,5 g Dimethylcarbamoylchlorid getropft. Das Gemisch wurde 3 Stunden unter Rückfluss gekocht. Nach dem Abkühlen, Abfiltrieren und Eindampfen wurde das Rohprodukt in 200 ml Chloroform gelöst und mit 100 ml Wasser ausgeschüttelt. Nach dem Abdestillieren des Lösungsmittels und überschüssiges Dimethylcarbamoylchlorid

erhielt man das 1-N,N-Dimethylcarbamoyl-3(5)-i-propyl-5(3)-(2-äthoxyäthylthio)-1,2,4-triazol (Verbindung Nr. 1) der Formel

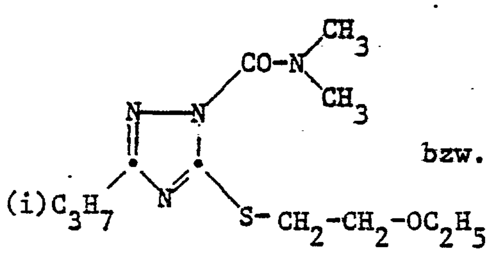

bzw.

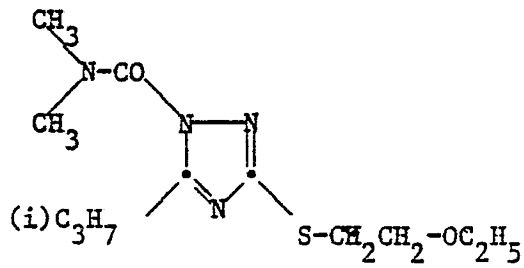

mit einem Brechungsindex von : $n_D^{20} = 1{,}5109$.

Die folgenden Verbindungen der Formel IA bzw. IB

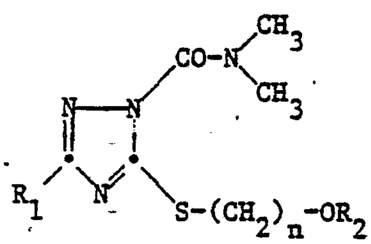

(IA)

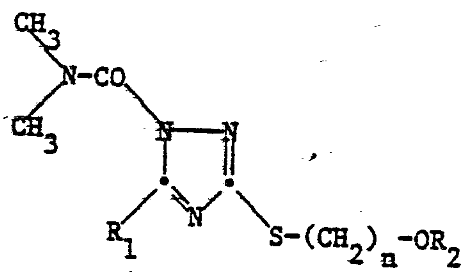

(IB)

können auf analoge Weise erhalten werden:

| Verb. Nr. | $R_1$ | $R_2$ | n | Physikalische Daten |
|---|---|---|---|---|
| 2 | $(i)C_3H_7$ | $CH_3$ | 1 | $n_D^{20}$ = 1,5198 |
| 3 | $(i)C_3H_7$ | $CH_3$ | 2 | $n_D^{20}$ = 1.5152 |
| 4 | $(i)C_3H_7$ | $C_2H_5$ | 1 | |
| 5 | $(t)C_4H_9$ | $CH_3$ | 2 | $n_D^{20}$ = 1.5102 |
| 6 | $(t)C_4H_9$ | $CH_3$ | 1 | F = 70°C |
| 7 | $(t)C_4H_9$ | $C_2H_5$ | 1 | |
| 8 | $(t)C_4H_9$ | $C_2H_5$ | 2 | $n_D^{20}$ : 1,5082 |
| 9 | $(t)C_4H_9$ | $(n)C_3H_7$ | 2 | |
| 10 | $(s)C_4H_9$ | $C_2H_5$ | 2 | |
| 11 | 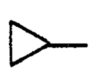 | $C_2H_5$ | 2 | $n_D^{20}$ : 1,5320 |
| 12 | 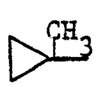 | $C_2H_5$ | 2 | $n_D^{20}$ = 1.5247 |
| 13 | 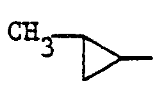 | $C_2H_5$ | 2 | |

Beispiel 2: Insektizide Frass- und Kontaktwirkung: Anthonomus grandis

Baumwollpflanzen wurden mit einer wässrigen Emulsion enthaltend 0,05% der zu prüfenden Verbindung (erhalten aus einem 25%igen Spritzpulver) besprüht.

Nach dem Antrocknen des Belages wurden die Pflanzen mit Adulten der Spezies Anthonomus grandis besiedelt. Man verwendete zwei Pflanzen für jede Versuchsverbindung und eine Auswertung der erzielten Abtötung erfolgte 2,4,24 und 48 Stunden nach Versuchsbeginn.

Der Versuch wurde bei 24°C und 60% relativer Luftfeuchtigkeit durchgeführt.

Verbindungen gemäss Beispiel 1 zeigten im obigen Versuch eine gute Wirkung gegen Insekten der spezies Anthonomus grandis.

Beispiel 3: Insektizide Frass- und Kontaktwirkung: Leptinotarsa decemlineata.

Bei gleicher Arbeitsweise unter Verwendung von Kartoffelstauden anstelle von Baumwollpflanzen und Larven der Spezies Leptinotarsa decemlineata im L3-Stadium, wurde die im Beispiel 2 beschriebene Versuchsmethode wiederholt.

Verbindungen gemäss Beispiel 1 zeigten ebenfalls in diesem Versuch eine gute Wirkung gegen Larven der Spezies Leptinotarsa decemlineata.

Beispiel 4: Insektizide Frass- und Kontaktwirkung: Aphis craccivora.

In Wasser angezogene Erbsen (Pisum sativum) wurden vor dem Versuchsbeginn je mit ca. 200 Individuen der Spezies Aphis craccivora besiedelt. Die so behandelten Pflanzen wurden 72 Stunden später mit einer Lösung enthaltend 200 oder 100 ppm der zu prüfenden Verbindung bis zur Tropfnässe besprüht.

Man verwendete pro Test-Verbindung und pro Konzentration zwei Pflanzen und eine Auswertung der erzielten Abtötungsrate der Blattläuse erfolgte nach weiteren 24 Stunden.

Verbindungen gemäss Beispiel 1 zeigten im obigen Versuch eine sehr gute Wirkung gegen Insekten der Spezies Aphis craccivora.

Beispiel 5: Insektizide Wirkung: Pseudococcus citri

In Töpfen angezogene Pflanzen (Vicia faba), welche bis auf ein gut ausgebildetes Blattpaar zurückgeschnitten war, wurden 24 Stunden vor dem Versuchsbeginn mit ca. 200 Individuen der Spezies Pseudococcus citri besiedelt. Am nächsten Tag wurden die nun mit Läusen besetzten Unterseite der Blätter mit einer Versuchslösung enthaltend 500 ppm der zu prüfenden Verbindung bis zu Tropfnässe besprüht. Man verwendete pro Test-Substanz zwei Pflanzen und eine Auswertung der erzielten Abtötung erfolgte 4 bis 24 Stunden nach Versuchsbeginn.

Verbindungen gemäss Beispiel 1 wirkten im obigen Versuch gegen Pseudococcus citri.

Beispiel 6: Insektizide Wirkung (systemisch): Aphis craccivora

Erbsenkeimlinge, die 24 Stunden vor Beginn des Versuches mit den Blattläusen infestiert worden waren, wurden in 20 ml einer wässrigen Brühe gestellt, die 12,5 ppm des zu prüfenden Wirkstoffes enthielt. Die wässrige Brühe wurde aus einem Emulsionskonzentrat oder

einer benetzbaren Pulverzubereitung des betreffenden Wirkstoffes hergestellt und befand sich in einem Gefäss, dass mit einem Löcher aufweisenden Plastikdeckel abgeschlossen war. Die Wurzel der infestierten Erbsenpflanze wurde jeweils durch ein Loch in dem Plastikdeckel in die Brühe geschoben. Das Loch wurde dann mit Watte abgedichtet, um die Pflanze zu fixieren und den Einfluss der Gasphase aus der Brühe auszuschalten.

Der Versuch wurde bei 20°C und 60% relativer Luftfeuchtigkeit durchgeführt. Nach zwei Tagen wurde auf die Anzahl der nicht mehr saugfähigen Testtiere, im Vergleich zu unbehandelten Kontrollen, bonitiert. Damit wurde festgestellt, ob der über die Wurzel aufgenommene Wirkstoff die Blattläuse an den oberen Pflanzenteilen abtötet.

Verbindungen gemäss Beispiel 1 zeigten im obigem Versuch eine gute systemische Wirkung gegen Insekten der Spezies Aphis craccivora.

<u>Beispiel 7:</u> Wirkung gegen pflanzenschädigende Akariden Tetranychus urticae (OP-sensible) und Tetranychus cinnabarinus (OP-tolerant)

Die Primärblätter von Phaseolus vulgaris Pflanzen wurden 16 Stunden vor dem Versuch auf akarizide Wirkung mit einem infestierten Blattstück aus einer Massenzucht von Tetranychus urticae (OP-sens.) oder Tetranychus cinnabarinus (OP-tol.) belegt. (Die Toleranz bezieht sich auf die Verträglichkeit von Diazinon).

Die so behandelten infestierten Pflanzen wurden mit einer Versuchslösung enthaltend 400 oder 200 ppm der zu prüfenden Verbindung bis zur Tropfnässe besprüht.

Nach 24 Stunden und wiederum nach 7 Tagen wurden Imagines und Larven (alle beweglichen Stadien) unter dem Binokular auf lebende und tote Individuen ausgewertet.

Man verwendete pro Konzentration und pro Testspezies eine Pflanze. Während des Versuchsverlaufs standen die Pflanzen in Gewächshauskabinen bei 25°C.

Verbindungen gemäss Beispiel 1 zeigen in diesem Versuch gute Wirkung gegen Individuen der Spezies Tetranychus urticae und Tetranychus cinnabarinus.

Patentansprüche

1. Verbindungen der Formel IA bzw. IB

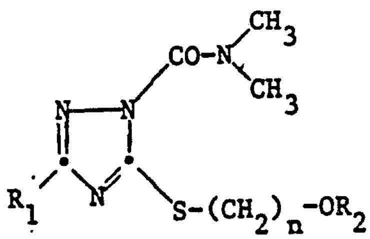

(IA)

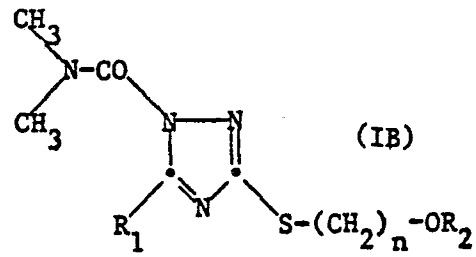

(IB)

worin $R_1$ eine i-Propyl-, s-Butyl-, t-Butyl- oder gegebenenfalls durch Methyl substituierte Cyclopropylgruppe, $R_2$ eine $C_1$-$C_3$-Alkylgruppe und n die Zahl 1 oder 2 bedeuten.

2. Verbindungen nach Anspruch 1, worin $R_1$ eine i-Propylgruppe, Cyclopropylgruppe oder t-Butylgruppe und $R_2$ eine Methyl- oder Aethylgruppe bedeuten.

3. Verbindungen nach Anspruch 2, worin $R_1$ eine i- Propylgruppe und $R_2$ eine Methylgruppe bedeuten.

4. Verbindungen nach Anspruch 3 der Formel

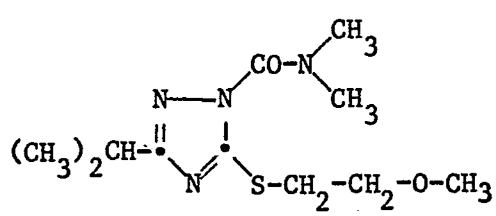

.

5. Verbindung nach Anspruch 3, der Formel

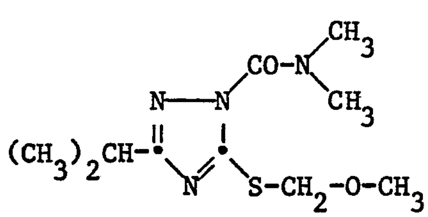

.

6. Verfahren zur Herstellung von im Anspruch 1 bis 5 definierten Verbindungen, dadurch gekennzeichnet, dass man eine Verbindung der Formel II

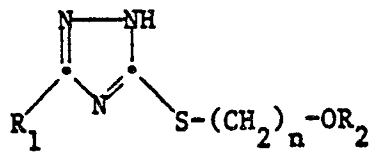

(II)

worin $R_1$, $R_2$ und n die entsprechenden im Anspruch 1 bis 3 angegebenen Bedeutungen haben, in Gegenwart einer Base mit einem N,N-Dimethylcarbamoylhalogenid umsetzt.

7. Schädlingsbekämpfungsmittel enthaltend als aktive Komponente eine Verbindung nach Anspruch 1 bis 5.

8. Verwendung einer Verbindung nach Anspruch 1 bis 5 zur Bekämpfung von Schädlingen.

9. Verwendung nach Anspruch 8 zur Bekämpfung von pflanzenschädigenden Insekten, insbesondere der Familie Aphididae.

10. Verbindungen der Formel II

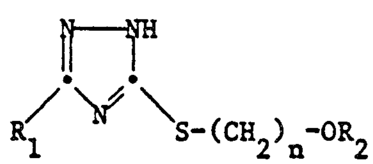

(II)

worin $R_1$, $R_2$ und n die in den Ansprüchen 1 bis 3 angegebenen Bedeutungen haben.

11. Verfahren zur Herstellung einer im Anspruch 10 definierten Verbindung, dadurch gekennzeichnet, dass man eine Verbindung der

Formel III

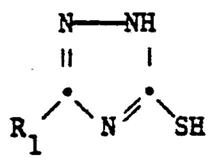

(III)

in Gegenwart einer Base mit einer Verbindung der Formel IV

$$Hal-(CH_2)_n-OR_2 \qquad (IV)$$

umsetzt, worin $R_1$, $R_2$ und n die im Anspruch 10 angegebenen Bedeutungen haben und Hal ein Halogenatom bedeutet.